Europäisches Patentamt

European Patent Office

Office européen des brevets

(11) Numéro de publication: **0 172 542**
**A2**

(12) **DEMANDE DE BREVET EUROPEEN**

(21) Numéro de dépôt: **85110299.6**

(22) Date de dépôt: **17.08.85**

(51) Int. Cl.⁴: **A 61 M 25/00**

(30) Priorité: **22.08.84 CH 4010/84**

(43) Date de publication de la demande:
**26.02.86 Bulletin 86/9**

(84) Etats contractants désignés:
**AT BE CH DE FR GB IT LI LU NL SE**

(71) Demandeur: **SARCEM SA**
**27 rue Cardinal-Journet Case Postale 371**
**CH-1217 Meyrin 1(CH)**

(72) Inventeur: **Jeanmonod, Maurice**
**47, Av. Vaudagne**
**CH-1217 Meyrin(CH)**

(72) Inventeur: **Bonello, Philippe**
**26, Chemin du Pommier**
**CH-1218 Grand-Saconnex(CH)**

(74) Mandataire: **Micheli, Michel-Pierre et al,**
**MICHELI & CIE 118, Rue du Rhône Case Postale 47**
**CH-1211 Genève 6(CH)**

(54) **Cathéter à commandes à distance.**

(57) La présente invention est un cathéter à commandes à distance destiné en principe à la chirurgie cardio-vasculaire, caractérisé par le fait qu'il permet à son utilisateur de commander directement, en partant de la poignée de l'instrument, l'orientation de sa tête et ceci au moyen d'un fil de commande (31) attaché à l'extrémité même de la tête du cathéter tout en étant décentré par rapport à l'axe de cette dernière d'une part, et à l'extrémité de l'arbre de traction (34) placé à l'intérieur de la poignée d'autre part, ce dernier pouvant se déplacer dans la direction de son axe et par conséquent tirer ou relâcher le fil de commande (31) lorsque l'utilisateur imprime un mouvement de rotation à la bague de commande (37) entraînant par sa denture intérieure le pignon intermédiaire (36) ce dernier engrènant à son tour dans la denture (35) de l'arbre de traction (34) qui possède également un filetage vis (38) travaillant avec un filetage écrou fixe (39) par rapport à la poignée.

La tête de ce cathéter comporte un ressort boudin cylindrique (28) coiffé d'une pièce de révolution à tête arrondie (29) appelée communément bouterolle, ces deux éléments au moins étant surmoulés d'un matériau (32) du genre caoutchouc de manière à réaliser une chambre étanche (33), est une construction qui permet d'obtenir par l'injection dans cette chambre (33) d'un fluide sous pression lui-même conjugué à la traction exercée sur le fil de commande (31), le raidissement de cette tête dans la forme recherchée tout en évitant son gonflement grâce aux spires du ressort boudin (28) pour les forces latérales du fluide et d'un fil (30) en U renversé passant par la fante de la bouterolle (29) et fixé par ses deux extrémités à la base du ressort (28) pour ce qui concerne les forces longitudinales. En ce qui concerne le ballon classique gonflé habituellement par un gaz, ce ballon pourrait être remplacé par une sorte de vessie 19 gonflable par un liquide en l'occurence un liquide de contraste introduit par le tuyau (4) et faisant suite dans la gaine (16) au travers de la chambre (14) pour s'échapper par les orifices (42) et (43) pratiqués dans cette gaine.

Pour ce qui est de la fonction bien connue d'introduire du liquide de contraste à l'extérieur de la tête de l'instrument et à l'intérieur du vaisseau, ce liquide sera injecté dans le tuyau 2 pour s'échapper ensuite par les deux orifices (24) et (25) du bâti (17) du présent cathéter.

./...

FIG. 1

La présente invention a pour objet un cathéter à tête orientable par action à distance dont les applications principales pourraient se situer dans le cadre des maladies cardio-vasculaires au niveau des vaisseaux coronaires, caractérisée par le fait qu'il permet à son utilisateur d'atteindre sans difficulté au travers des multiples ramifications circulatoires la zone à traiter tout en ayant la possibilité également par action à distance de provoquer le raidissement de sa tête pendant le temps nécessaire et ceci à n'importe quel moment de l'intervention chirurgicale.

Le dessin annexé représente à simple titre d'exemple et non limitatif, une forme d'exécution dans son principe de l'objet de la présente invention.

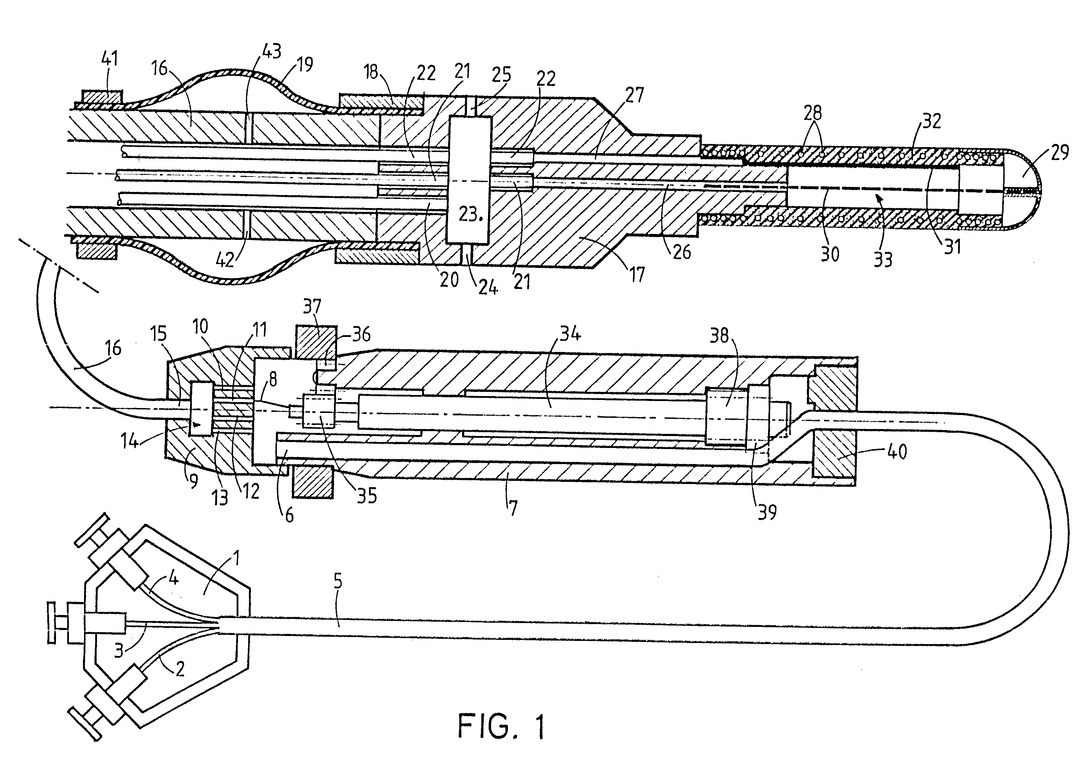

La figure 1 en est une vue générale en coupe.

Le cathéter à commandes à distance représenté au dessin comprend une prise d'alimentation 1 à trois tuyaux 2;3 et 4 réunis ensemble dans une gaine 5 dont l'une de ses extrémités est fixée à la sortie de la prise d'alimentation 1 alors que la seconde de ses deux extrémités coïncide avec la fin du trou cylindrique 6 pratiqué dans le manche 7 de la poignée du cathéter.

Aux trois tuyaux 2;3 et 4 dénudés de leur gaine 5 prend naissance sensiblement à la hauteur de la sortie du trou cylindrique 6 un tuyau supplémentaire 8. La tête 9 de la poignée possédera donc quatre canaux 10;11;12 et 13 se terminant à l'entrée de la chambre 14 et destinés à positionner et à fixer par serrage les quatre tuyaux 2;3;4 et 8. Par ailleurs et entre la chambre 14 et l'extrémité de la tête 9 de la poignée est prévu un cinquième canal 15 de plus fort diamètre dont le rôle est de positionner et fixer également par serrage l'extrémité d'une gaine 16 contenant les tuyaux 8;2 et 3, le tuyau 4 se terminant à l'entrée de la

- 2 -

chambre 14.

A l'autre extrémité de la gaine 16 et dans sa prolongation immédiate est fixé le bâti 17 de la tête du cathéter au moyen de la bague de serrage 18 coiffant les deux éléments dont la seconde fonction est de positionner rigidement et du même coup l'une des deux parties extrêmes de la vessie 19 sur la gaine 16 et le bâti 17 tout en garantissant entre la gaine 16 et le bâti 17 d'une part et la vessie 19 d'autre part l'étanchéité nécessaire au bon fonctionnement de l'instrument.

En examinant le bâti 17 de la tête du cathéter, on remarquera que ce dernier possède trois canaux 2;21 et 22 destinés à positionner et à fixer par serrage les trois tuyaux 8;2 et 3 et débouchant sur une chambre de distribution 23 prolongée de deux trous latéraux 24 et 25 donnant sur l'extérieur. Seuls les canaux 21 et 22 allant au-delà de la chambre 23 en sont prolongés de deux plus petits de diamètre numérotés 26 et 27.

Quant à la tête du cathéter proprement dit, cette dernière est composée d'un ressort cylindrique 28 dont la base est enmanchée sur le bâti 17, une bouterolle 29 dont le tige-rond est enmanché sur l'autre extrémité du ressort 28, d'un fil 30 en forme de U renversé représenté sur le dessin en pointillé passant au travers de la fente pratiquée dans la tête de la bouterolle 29 et soudé par ses deux extrémités sur la base du ressort 28, d'un fil de commande 31 soudé par son extrémité sur le tige-rond de la bouterolle 29 ce fil étant excentré par rapport à l'axe de cette dernière, le tout surmoulé d'un matériau 32 type caoutchouc de manière à obtenir une chambre 33 dont le rôle est d'être à même de contenir un fluide

0172542

- 3 -

sous une certaine pression.

Pour diriger la tête du cathéter dans l'origine de l'une ou l'autre des ramifications circulatoires choisies, l'utilisateur dispose pour actionner son instrument de trois degrés de liberté.

Avance ou recul de l'ensemble et par conséquent de sa tête, rotation de l'ensemble sur lui-même dans un sens ou dans l'autre par rapport à son axe, inclinaison de la tête uniquement grâce à la force de traction que l'utilisateur imprimera sur le fil de commande 31 gainé par le tuyau 8 le fil 31 étant attaché par son extrémité à celle de l'axe de traction 34 possédant une partie dentée 35 engrènant avec un pignon intermédiaire 36 lui-même engrènant à son tour avec la denture intérieure de la bague de commande 37 accessible directement par l'utilisateur.

Pour que l'axe de traction 34 recule lorsque l'utilisateur imprime un mouvement de rotation à la bague de commande 37, il est nécessaire que cet axe 34 possède un filetage 38 engrènant dans un filetage fixe 39 les deux fonctions principales du bouchon 40 étant de jouer le rôle de butée à l'axe 34 d'une part et de maintenir centrée et en place la gaine 5 d'autre part.

Si le raidissement de la tête du cathéter existe déjà et dans une très faible mesure par l'équilibre établi entre la force intrinsèque du ressort cylindrique 28 et la force de traction exercée sur le fil de commande 31 , il est possible d'amplifier ce raidissement en injectant par le tuyau 3 sous une certaine pression, un fluide à l'intérieur de la chambre 33 dont les spires du ressort cylindrique 28 forment une armature aux forces latérales développées par le fluide, le fil 30 en forme de U renversé constituant à son tour une armature aux forces longitudinales empêchant un allongement de la chambre 33 donc de la tête du cathéter. D'autre part, et pour le meilleur mouvement d'inclinaison de la tête, le plan formé par le fil 30 et le plan formé par le fil de commande 31 avec l'axe du ressort cylindrique 28 seront orthogonaux entre eux.

En ce qui concerne le ballon glonfable au moyen d'un gaz pour les cathéters classiques et bien connus, cette fonction dont le but thérapeutique est le même se voit remplacé dans l'instrument faisant l'objet de la présente invention par une sorte de vessie 19 gonflable au moyen d'un fluide introduit par le tuyau 4 et pénétrant dans la gaine 16 pouvant être en l'occurence un liquide de contraste pratiquement incompressible permettant outre des commandes immédiates d'être lu facilement en examens radioscopiques

Par ailleurs et en cas de fuite, l'innocuité du liquide contrastant doit être considéré comme un critère de la plus haute importance. Inutile de préciser que l'étanchéité nécessaire entre l'autre partie extrême de la vessie 19 et la gaine 16 est assurée selon le principe déjà décrit par une seconde bague de serrage 41.

Le liquide nécessaire au remplissage de la vessie 19 est injecté par le tuyau 4 envahissant totalement la chambre 14 pour s'introduire ensuite dans la gaine 16 entre les tuyaux 2;3 et 8 pour s'échapper finalement par les trous latéraux 42 et 43 pratiqués sur cette gaine 16 et situés sensiblement à mi-longueur de la vessie 19.

Reste la fonction classique et bien connue consistant à introduire du liquide de contraste à l'intérieur de la ramification circulatoire considérée

- 5 -

et ceci à des fins de repère pour lecture en examens radioscopiques. Pour celà, le liquide contrastant est injecté par le tuyau 2 envahissant la chambre 23 et s'échappant à l'extérieur dans la ramification par les deux orifices 24 et 25 du présent cathéter.

REVENDICATIONS

1.      Cathéter à commandes à distance, caractérisé par le fait que sa tête peut être orientée au moyen d'un fil de commande.

2.      Cathéter à commandes à distance, caractérisé par le fait qu'il est possible de provoquer le raidissement de sa tête en soumettant cette dernière à la pression d'un fluide qui lui est en partie intérieur.

3.      Cathéter à commandes à distance, caractérisé par le fait que le ballon classique gonflable au moyen d'un gaz est remplacé par une sorte de vessie gonflable au moyen d'un liquide et plus particulièrement par un liquide de contraste.

4.      Cathéter à commandes à distance, caractérisé par le fait que le liquide de contraste rejeté à l'extérieur et destiné à envahir en tout ou partie l'intérieur du vaisseau à contrôler est éjecté ici par un ou plusieurs orifices latéraux pratiqués dans un élément relativement dur appelé bâti servant de support à la tête du cathéter.

5.      Cathéter à commandes à distance, caractérisé par le fait que sa tête est formée en partie d'un ressort boudin cylindrique dont l'une de ses extrémités est enmanchée sur le bâti servant de support à la tête du cathéter.

6.      Cathéter à commandes à distance, caractérisé par le fait que l'autre extrémité du ressort boudin cylindrique est enmanchée sur le tige-rond d'une pièce de révolution à tête arrondie.

7.      Cathéter à commandes à distance selon la revendication 1, caractérisé par le fait que le fil de commande est fixé à l'extrémité de la tête et décentré par rapport à l'axe de cette dernière.

8.      Cathéter à commandes à distance selon revendications 1;6 et 7, caractérisé par le fait que le fil de commande est soudé sur un méplat du tige-rond de la pièce de révolution à tête arrondie.

9.      Cathéter à commandes à distance selon revendications 6 et 8, caractérisé par le fait que la tête arrondie de la pièce de révolution est fendue latéralement de part en part.

10.      Cathéter à commandes à distance selon revendications 2;5;6;8 et 9, caractérisé par le fait que le ressort boudin cylindrique et la pièce de révolution à tête arrondie tous les deux au moins sont surmoulés d'un matériau genre caoutchouc de manière à obtenir à l'intérieur de la tête du cathéter une chambre capable de pouvoir supporter un fluide liquide ou gazeux sous une certaine pression.

11.      Cathéter à commandes à distance selon revendications 2;5;6;8;9 et 10, caractérisé par le fait que la tête du cathéter est entourée dans le sens de sa longueur par un fil dont les extrémités sont fixées diamétralement et en principe sur la base extérieure du ressort boudin cylindrique passant de préférence à l'intérieur des spires du ressort et le plus près possible de ces dernières ainsi qu'entre les spires

- 8 -

enroulées sur le tige-rond de la pièce de révolution à tête arrondie et ce dernier possédant deux méplats diamétralement opposés à cet effet.

Par ailleurs, la fente latérale pratiquée dans la tête de forme arrondie est nécessaire au passage et au maintien du fil en bonne position.

12. Cathéter à commandes à distance selon revendications 1;7 et 8, caractérisé par le fait que le fil de commande est fixé par ailleurs à un arbre de traction placé à l'intérieur de la poignée du cathéter.

13. Cathéter à commandes à distance selon revendication 12, caractérisé par le fait que l'arbre de traction possède sur son pourtour une denture engrènant avec un pignon intermédiaire ce dernier engrènant à son tour avec la denture intérieure d'une bague de commande dont l'axe de rotation se confond avec celui de la poignée.

14. Cathéter à commandes à distance selon revendications 12 et 13, caractérisé par le fait que l'arbre de traction possède un filetage vis travaillant avec un filetage écrou fixe par rapport à la poignée de telle manière que sa rotation sur lui-même et autour de son axe lui imprime un mouvement axial dont l'effet est une traction ou au contraire un relachement du fil de commande selon le sens de rotation donné à cet arbre de traction.

15. Cathéter à commandes à distance selon revendications 1;7;8;9 et 11, caractérisé par le fait que le plan formé par le fil de commande et l'axe de la tête d'une part et le plan formé par le fil entourant la tête dans le sens de sa longueur d'autre part sont

- 9 -

orthogonaux entre eux.

16.	Cathéter à commandes à distance selon revendications 4 et 5, caractérisé par le fait que son bâti est formé de deux parties destinées à être montées ensemble en "sandwich" dont l'axe commun se confondant avec celui de la tête se trouve être dans le plan de joint des deux parties.

17.	Cathéter à commandes à distance selon revendications 2;3 et 10, caractérisé par le fait que les circuits pneumatiques ou hydrauliques sont équipés de dispositifs de sécurité genre soupapes, ces derniers étant situés hors de la partie de l'instrument réservée à être introduite tout ou partie à l'intérieur du corps à traiter.

18.	Cathéter à commandes à distance selon revendications 1;2;3;4 et 16, caractérisé par le fait que les fixations des différents tuyaux tant au niveau de la prise d'alimentation et de la poignée qu'à celui de la tête du cathéter se font en tout ou partie par serrage de ceux-ci dans leurs canaux correspondants en empreintes négatives dans chacune des parties destinées à être montées en "sandwich".

19.	Cathéter à commandes à distance selon revendications 3 et 16, caractérisé par le fait que les deux parties du bâti sont assemblées et fixées rigidement ensemble l'une à l'autre au moyen de bagues fendues de telle manière que leurs extrémités se chevauchant puissent être soudées l'une sur l'autre électriquement par points, ce système à bagues pouvant être également appliqué à la fixation vessie ballon poignée ou prise d'alimentation du cathéter.

20.    Cathéter à commandes à distance selon revendications 12;13 et 14, caractérisé par le fait que la poignée est composée entre autre d'un manche et d'une tête de poignée, ces deux pièces étant fixées l'une à l'autre de telle manière que la bague de commande située entre deux soit libre circonférentiellement tout en étant maintenue par un minimum de jeu en bonne position axiale.

21.    Cathéter à commandes à distance selon revendications 16;18 et 19, caractérisé par le fait que l'une ou l'autre ou plusieurs parties de ce cathéter destinées à être montées ensemble en "sandwich" peuvent être, baguées ou non, soumises à un traîtement de soudage par ultra-son.

22.    Cathéter à commandes à distance selon revendications 1;5;6;7;8 et 12, caractérisé par le fait que le fil de commande surmoulé à la pièce en principe de révolution ressort axialement par son autre extrémité au tige-rond pour être ancré à son bâti.

23.    Cathéter à commandes à distance selon revendication 22, caractérisé par le fait que le fil de commande et le fil qui sort axialement au tige-rond ne forment pas qu'un seul et même fil mais deux fils différents.

24.    Cathéter à commandes à distance selon revendications 22 et 23, caractérisé par le fait que le ou les fils ne sont pas surmoulés à la pièce en principe de révolution mais fixés à elle par un moyen différent.

FIG. 1